**Europäisches Patentamt**

**European Patent Office**

**Office européen des brevets**

(11) Publication number : **0 561 489 A2**

# EUROPEAN PATENT APPLICATION

(21) Application number : 93300458.2

(22) Date of filing : 22.01.93

(51) Int. Cl.$^5$ : **A61K 7/32,** C07C 37/08, C07C 409/10, C07C 7/148

(30) Priority : 22.01.92 US 823777

(43) Date of publication of application :
22.09.93 Bulletin 93/38

(84) Designated Contracting States :
AT BE CH DE DK ES FR GB IE IT LI LU NL SE

(71) Applicant : THE MENNEN COMPANY
Hanover Avenue
Morristown New Jersey 07962-1928 (US)

(72) Inventor : Barr, Morton Lawrence
4 Wenonah Avenue
Rockaway, New Jersey (US)

Inventor : Vojt, Christine Mary
14 Laurie Avenue
Hopatcong, New Jersey (US)
Inventor : Vincenti, Paul Joseph
95 Minnisink Road
Jefferson, New Jersey (US)
Inventor : Balish, Edward
2 Shiloh Court
Madison, Wlnconsin (US)
Inventor : Vanderhoof, Elaine Lucatelli
125 Schooleys Mountain Road
Long Valley, New Jersey (US)

(74) Representative : Kearney, Kevin David
Nicholas et al
KILBURN & STRODE 30 John Street
London, WC1N 2DD (GB)

(54) Deodorant compositions containing materials for inhibiting bacterial adherence, method of use thereof, and method for determining materials that inhibit bacterial adherence.

(57)   Disclosed are deodorant compositions, including deodorant compositions for axillary regions and body lotions (e.g., baby lotions), which can reduce body malodor (such as malodor arising in axillary regions of the human body) without use of antimicrobial agents. The compositions include a material (e.g., selected from specific glycoproteins and carbohydrates) which inhibits adherence of malodor-causing bacteria to the skin, wherein malodor-causing bacterial populations, and the malodor caused by such populations, are reduced. Also disclosed is an analytical technique, using differentiated human epithelial cells, which can be used to determine the ability of tested materials to inhibit adherence of malodor-causing bacteria to human skin surfaces.

EP 0 561 489 A2

TECHNICAL FIELD

The present invention is directed, most generally, to compositions for application to the skin surface to inhibit and reduce adherence of odor-causing bacteria (in particular, malodor-causing bacteria) to the skin, which compositions do not require antimicrobial agents.

More specifically, the present invention relates to deodorant compositions, for application to the skin, such as to axillary regions or other body regions of the human body, to reduce (prevent) body malodor. In particular, the present invention is directed to lotions (e.g., body lotions, including baby lotions), or axillary deodorant compositions for use in the form of a roll-on, aerosol, stick, suspension, etc., which avoids malodor without requiring an antimicrobial agent.

In addition, the present invention is directed to an analytical method for determining efficacy of various materials to reduce adherence of bacteria (e.g., malodor-causing bacteria) to the skin (for example, the skin of a person, including the skin in axillary regions of the body).

BACKGROUND ART

It has long been desired to avoid body malodor (for example, underarm malodor or malodor caused by residual body wastes on the skin of a baby). There are many mechanisms of deodorancy known in the art. For example, odor masking, involving use of a composition including a fragrance to simply overpower the body malodor, may be utilized. Other deodorant compositions, for avoiding body malodor, include components which reduce the partial vapor pressure of morpholine. These same disclosed compositions include components to achieve a significant lipoxidase inhibition, in order to prevent oxidation of unsaturated compounds to malodorous substances.

Other disclosed deodorant compositions include alkaline metal bicarbonates, the bicarbonates acting to chemically neutralize odoriferous short-chain fatty acids in, e.g., the axillary regions of the body. Also known is a method of deodorancy utilizing zinc glycinates, thought to act by both chemical neutralization of malodorous chemicals and by inhibiting bacterial growth.

The use of antimicrobial agents to inhibit growth of bacteria (e.g., axillary bacteria), and therefore decrease formation of malodor caused by these bacteria, is well known. Initially, hexachlorophene, and later triclocarban, triclosan and several quaternary ammonium compounds such as benzethonium chloride and methyl benzethonium chloride have been used in commercial deodorant products. Also known are anhydrous deodorant compositions containing triacetin, or 1,2,3-propane triacetate. Similarly, triethyl citrate and esters of hydroxy carboxylic acid have been disclosed for use in deodorant products. However, deodorant compositions including antimicrobial agents disadvantageously affect the microorganism environment on the skin. Moreover, various antimicrobial agents set forth above, such as hexachlorophene, have been banned in the past as unsafe.

It is also known to use ethylenediaminetetraacetic acid (EDTA), and other metal sequestering agents, as deodorant active ingredients, which act to remove critical metal nutrients required by bacteria for enzymic formation of free acids.

U.S. Patent No. 4,518,517 to Eigen, et al., the contents of which are incorporated herein by reference in their entirety, discloses deodorant body cleansing compositions, utilizing a still further technique for reducing (or avoiding) malodor. Specifically, this patent discloses that the number of odor-causing bacteria on the skin can be reduced by using an effective amount of specific sugars (mannose, glucose and oligomers thereof) that interfere with the mechanism by which the odor-generating bacteria adhere to the skin. This U.S. patent discloses that the addition of about 1-15% by weight of a carbohydrate selected from the group consisting of glucose, mannose, oligomers thereof and a mixture thereof, to a body cleansing composition produces a deodorant composition which reduces the odor-causing microbial population on the skin, without the use of antimicrobials.

This U.S. Patent No. 4,518,517 discloses that mannose, glucose and oligomers thereof, and mixtures thereof, have specificity to lectins of the odor-causing bacterial organisms, whereby body odors are reduced by detachment of the odor-causing organisms from the skin. Thus, this patent discloses that lectin technology (lectins being proteins or glycoproteins which have an affinity for specific sugars and which are present in the cell membranes of bacteria and are one mechanism by which they adhere to surfaces) can be used as a means of reducing numbers of odor-causing bacteria on the skin.

This U.S. Patent No. 4,518,517 also discloses a test procedure, used in connection with various sugars, to determine whether such sugars all inhibit bacterial attachment to the skin. The test procedure utilized human stratum corneum, blended in a physiological saline solution. Various sugars to be tested were incorporated into different samples of the blend of human stratum corneum and saline solution. Thereafter, a bacterial suspension, made by suspending small colony diphtheroids into a sterile saline solution, was added to each of the different samples containing the sugar to be tested and the blend of human stratum corneum and saline solution.

This U.S. Patent No. 4,518,517 discloses that neither galactose nor N-acetyl glucosamine had an effect on the diphtheroid bacterial population on the skin, in the described test; that mannose was the only sugar that showed inhibition of bacterial attachment, as shown by fewer bacteria attached to the skin sample; and that additional tests made with other sugars including fructose, lactose, maltose, sucrose, raffinose and rhamnose showed a greater amount of bacteria attached to the skin than one treated with mannose, thereby proving the specificity of mannose to the diphtheroid bacteria. In using the specific testing technique described in U.S. Patent No. 4,518,517, this patent discloses that of various carbohydrates only mannose, glucose and oligomers thereof (and mixtures thereof) can be used to inhibit adherence of odor-generating bacteria to the skin.

This U.S. Patent No. 4,518,517 further discloses that mannose, glucose, oligomers thereof, and mixtures thereof, can be used in deodorant detergent compositions containing an anionic water solubilizing group, and further conventional additional components (such as coloring agents and perfumes); and that the composition can be in a liquid form, or opaque deodorant detergent bar (solid) form.

Use of other materials, for inhibiting adherence of specific bacteria to various specific surfaces, using various specific adherence-restricting agents, is known. Thus, the article in Nature, Vol. 365 (September 17, 1977), pages 623-5, discloses that D-mannose and methyl-α-D-mannopyranoside inhibited adherence of different strains of Escherichia coli to human epithelial cells, but did not inhibit adherence of streptococci to such epithelial cells. Tojo, "Isolation and Characterization of a Capsular Polysaccharide Adhesin from Staphylococcus Epidermidis" in The Journal of Infectious Diseases, Vol. 157, No. 4 (April 1988), pages 713-721 discloses a polysaccharide adhesin isolated from a specific strain of the Staphylococcus epidermidis; this article discloses that the adhesin was composed of a complex mix of monosaccharides (with galactose and glucosamine predominating) and bound well to silastic catheter tubing while inhibiting adherence of the specific strain to catheters.

U.S. Patent No. 4,737,359 to Eigen, et al. discloses a method for inhibiting dental plaque and promoting oral hygiene, including dispersing a plaque-inhibiting amount of emulsan in water and contacting the aqueous dispersion of emulsan with natural or artificial dental surfaces. This U.S. Patent No. 4,737,359 discloses that the use of the emulsan reduces plaque formation through inhibition of attachment of Streptococcus mutans (which is a prime factor in causing plaque formation on teeth) to dental surfaces.

As seen in the foregoing, although compounds inhibiting adherence of various specific microorganisms to specific surfaces are known, apart from U.S. Patent No. 4,518,517 none of the aforementioned disclosures show deodorant compositions for inhibiting malodor-causing bacteria from adhering to the skin of a person, particularly to the skin in axillary regions.

Moreover, U.S. Patent No. 4,518,517, using the specific testing techniques described therein, discloses that only mannose, glucose, oligomers thereof, and mixtures thereof, can be used to inhibit adherence of the tested odor-causing bacteria to the skin, and that other sugars do not inhibit adherence of such bacteria.

U.S. Patent No. 4,263,274 to Kulkarni, et al. discloses compositions and methods for inhibiting perspiration, such compositions including an aldehyde polysaccharide compound, so that effective perspiration control may be achieved without use of metallic anhidrotic agents. This U.S. Patent No. 4,263,274 discloses antiperspirant compositions including aldehyde polysaccharide compounds; however, this patent does not disclose any inhibition of adherence of odor-causing bacteria to the skin when using the described aldehyde polysaccharide compounds, and does not disclose use of aldehyde polysaccharide compounds for deodorancy apart from an antiperspirant use.

U.S. Patent No. 4,822,596 to Callingham, et al. discloses an antiperspirant composition having a non-aqueous liquid phase and a solid phase, the solid phase including a moisture-absorbent organic polymer (such organic polymers, as disclosed, include various polysaccharides (poms)) in particulate form. Clearly, the organic polymer in U.S. Patent No. 4,822,596, part of an antiperspirant composition, acts to absorb moisture, and is applied in particulate form so that the organic polymer is deposited on the skin in powder form. This patent does not disclose that the organic polymer can act to prevent adherence of malodor-causing bacterial to the skin, particularly in axillary regions.

Accordingly, it is desired to provide a deodorant composition, operating by the technique of inhibiting adherence of odor-causing bacteria to the body (for example, to skin of the body, particularly in the axillary region or in regions where residual body wastes are on a baby), utilizing compounds other than mannose, glucose and oligomers thereof, to provide such deodorant compositions in various forms for use, and to provide methods of using such deodorant compositions. Moreover, it is desired to provide such deodorant compositions, utilizing such agents for inhibiting adherence of malodor-causing bacteria to the skin, and (1) avoiding antimicrobial agents in the composition (that is, to provide deodorant compositions which do not contain antimicrobial agents), and (2) avoiding high- or low-pH compositions (which can be irritating). Moreover, it is also desired to provide such deodorant composition, without the necessity of incorporating a fragrance in the deodorant (such fragrance masking body malodor).

## DISCLOSURE OF INVENTION

Accordingly, it is an object of the present invention to provide a composition, and method of using such composition, which can reduce adherence of malodor-causing aerobic and anaerobic bacteria to the skin, particularly in various regions of the human body (including axillary regions of the human body and body regions of a baby). By reducing adherence to the skin, it is meant that less bacteria attach to the skin surface, after application of the composition to the skin surface, than would attach to the skin in the absence of such composition.

It is a further object of the present invention to reduce body malodor caused by bacteria on the skin, particularly in various regions of the body, both in adults and babies.

It is a further object of the present invention to provide a deodorant composition, and method of using such composition, to reduce body malodor without use of antimicrobial agents in the deodorant composition.

It is a still further object of the present invention to provide a deodorant composition, and method of using such composition, to reduce body malodor by reducing adherence of malodor-causing bacteria on skin (human skin, for example), including in axillary regions, and on the skin of both adults and babies.

It is a further object of the present invention to provide a deodorant composition, and method of using such composition, which does not require a fragrance, and which need not have a high or low pH (thereby avoiding skin irritation caused by applying such high- or low-pH material to the skin).

It is a still further object of the present invention to provide deodorant stick compositions for reducing body malodor without the use of antimicrobial agents in the composition.

It is a still further object of the present invention to provide lotion compositions, particularly baby lotion compositions having the necessary gentleness to be used on a baby, which reduces malodor without the use of antimicrobial agents and without incorporating a fragrance in the composition (although the antimicrobial agent and/or fragrance can be incorporated in the composition).

It is a further object of the present invention to provide an analytical method for determining whether specific materials (e.g., carbohydrates and proteins) reduce adherence of malodor-causing bacteria to skin, indicative of whether such materials reduce adherence of malodor-causing bacteria to the skin, for example, in axillary regions of the body.

The present invention achieves these objects with compositions incorporating carbohydrates and/or proteins, including monosaccharides and polysaccharides, polymer carbohydrates, and oligomers and derivatives of the carbohydrates, and mixtures thereof. Contrary to the teachings of U.S. Patent No. 4,518,517, various carbohydrates, other than those described in U.S. Patent No. 4,518,517, can be used as part of the present invention, to inhibit adherence of malodor-causing bacteria to the skin; that is, adherence-inhibiting carbohydrates are not limited to mannose, glucose and oligomers thereof, and mixtures thereof, as described in U.S. Patent No. 4,518,517. In fact, various of the carbohydrates and proteins used as part of the present invention provide even better results, in inhibiting adherence of malodor-causing bacteria to skin of a human body, than mannose, glucose and oligomers thereof, and mixtures thereof as described in U.S. Patent No. 4,518,517.

The results according to the present invention are particularly surprising in light of the disclosure in U.S. Patent No. 4,518,517, as well as general knowledge in the art. As described previously, U.S. Patent No. 4,518,517 discloses that, from the test procedures described therein, only certain specific sugars, and oligomers thereof, and mixtures thereof, act to inhibit adherence of certain bacteria to the skin, and other sugars do not inhibit adherence of the same bacteria to the same surfaces. Moreover, it is known, as seen in various of the patents and published articles discussed previously, that specific compounds can inhibit adherence of specific bacteria to specific surfaces, but those same compounds will not necessarily act to inhibit adherence of other bacteria to other surfaces; different bacteria use different mechanisms for adherence, so that the effect of a compound on adherence of a type of bacteria to one surface will not necessarily be the same as that of the same compound on a different type of bacteria on another surface. Notwithstanding this knowledge in the art, applicants have found classes of compounds that inhibit adherence of malodor-causing bacteria to the skin, so as to achieve the objectives of the present invention.

Specific carbohydrates (e.g., simple carbohydrates, i.e., monosaccharides) which can be used according to the present invention, as part of deodorant compositions to inhibit adherence of malodor-causing bacteria, include galactose, N-acetyl-D-galactosamine, fucose, N-acetyl-D-glucosamine, D-glucuronic acid, and derivatives (compounds containing the monosaccharide and a group added to the monosaccharide (e.g., by a simple chemical process), yet which retain a structural radical of the monosaccharide) and oligomers (oligosaccharides) of these carbohydrates or oligomers containing these carbohydrates. The monosaccharides need not be water-soluble , but of course they can be. By oligomers, we mean a compound containing a plurality (e.g., up to 10) of monosaccharides joined together by acetal-type linkages.

Moreover, polymer carbohydrates (polysaccharides) which can be used according to the present invention include emulsan and xanthum gum, alginic acid, alginates (for example, sodium and potassium salts of alginic acid), guar gum, locust bean gum, gellam gum, various forms of carageenans, and fragments thereof. By polymer carbohydrates (polysaccharides), we mean a compound containing a large number (e.g., at least ten (10)) monosaccharides joined together by acetal-type linkages. By fragments, we mean, e.g., hydrolysis products or products of hydrolytic cleavage of polysaccharides.

Such fragments are achieved by well known enzymatic, chemical and thermal treatment of these polymer carbohydrates. For example, fragments of the aforementioned polymer carbohydrates may be formed by hydrolysis by heating solutions of 1-5 percent by weight in water to 80-100°C, for up to 6 hours. The presence of a catalyst such as inorganic acid (e.g., HC1) or base (e.g., NaOH) may be necessary. The pH range may vary between 2-11, depending upon the specific polymer carbohydrate. The purpose of hydrolysis is to reduce the size and the molecular weight of the polymer carbohydrate, and to reduce the viscosity of the aforementioned 1-5 percent solutions.

Alternatively, enzymatic degradation as practiced by U.S. Patent No. 4,818,817 may be useful to provide the fragments. In this U.S. patent, a microbial enzyme capable of cleaving glycosidic bonds is disclosed as a useful way of fragmenting emulsans.

The specific viscosity or molecular size of the fragments are not important, as both fragments and whole polymer carbohydrates are effective in inhibiting adhesion of malodor-causing bacteria to the skin.

Of the foregoing polymer carbohydrates, the alginates are preferred commercially, both for deodorant compositions for axial regions of the human body and for body lotions (including baby lotions) that inhibit bacterial adherence to the skin. It is desired that the alginates, as part of the compositions according to the present invention, be hydrolyzed to make them less viscous and easier to use in manufacturing the deodorant composition according to the present invention.

The polymeric carbohydrates (gums), as part of the deodorant composition according to the present invention, are dispensed in soluble or partly soluble form as part of the roll-on composition, stick composition, aerosol composition, lotion, etc. This is clearly different, and achieves an entirely different result, than the powder-containing compositions of U.S. Patent No. 4,822,596. For example, in powder form the polymeric carbohydrates of the compositions of U.S. Patent No. 4,822,596 are unable to interact with and bind to skin or bacterial cells, and are thus unable to inhibit adherence of bacteria to skin.

Although the water-soluble, powdered, polymeric carbohydrates in the compositions of U.S. Patent No. 4,822,596 may solubilize in underarm moisture (perspiration), this is a slow process; first, the powder absorbs small amounts of moisture without dissolving, and thereafter small amounts of powder may dissolve in excess underarm perspiration, but the solubilized polymer carbohydrate is trapped in the thick paste first formed. Considerable time (perhaps hours) is required for any dissolved polymer to become free to interact with bacteria or skin cells, if indeed, the dissolved polymer ever becomes free. However, deodorant efficacy requires immediate or at least rapid availability of the active deodorant ingredient. Furthermore, some of the polymer carbohydrates (e.g., carageenans) require considerable thermal and/or mechanical energy to effect dissolution into water; such conditions do not exist on the human body surface. From the foregoing, it can be appreciated that compositions containing polymer carbohydrates in powder form, as in U.S. Patent No. 4,822,596, will not achieve the objectives of the present invention.

As can be appreciated from the foregoing, the bacteria adherence-inhibiting agent desirably should be water-soluble, and in a form so as to be easily solubilized in contact with, e.g., perspiration or urine.

As proteins which can be utilized as a component of deodorant compositions for inhibiting adherence of malodor-causing bacteria to the skin of a body, according to the present invention, are glycoproteins (conjugated proteins containing a carbohydrate radical and a simple protein); such glycoproteins include laminin, fibronectin, chondronectin, collagen, and a-acid glycoprotein from any one of several sources, including bovine sources. Generally, glycoproteins from human and animal sources inhibit adhesion of malodor-causing bacteria from axillary regions.

It is important to note that not all proteins are useful as a component of deodorant compositions for inhibiting adherence of malodor-causing bacterial to the skin of a body. For example, wheat germ aglutin (Triticum Vulgaris) and jack bean lectin (Concanavalin A) both promote the adhesion of malodor-causing bacteria to the skin. However, various proteins (e.g., various glycoproteins) which can inhibit adherence of malodor-causing bacteria to the skin of a body can easily be determined, particularly in view of the testing procedure, for determing adherence-inhibiting materials, discussed further infra. In the following, reference to proteins, or to glycoproteins, as part of the deodorant composition of the present invention is to those proteins or glycoproteins, respectively, that inhibit adherence of malodor-causing bacteria to skin of the human body, including to skin in axillary regions.

In the composition according to the present invention, the adherence-inhibiting polymeric carbohydrates are desirably at least partly in solution (e.g., in an aqueous solution) when applied (that is, the adherence-inhibiting polymeric carbohydrates are dispensed in soluble or partly soluble form from any one of several compositions, e.g., stick, aerosol, liquid, lotion, etc.). There is no solubility restriction where the adherence-inhibiting material is a simple sugar or glycoprotein, although each may be at least partly in solution. As for the various oligomers that fall within the scope of the present invention, as the molecular weight of the oligomer used is larger, it becomes more preferable that the adherence-inhibiting carbohydrate be at least partly in solution when applied.

Desirably, the carbohydrate or protein incorporated in the composition is utilized in amounts effective to so inhibit adherence of malodor-causing bacteria as to reduce the malodor-causing bacterial population on the skin. Illustratively, the carbohydrate and/or protein is incorporated in the composition in amounts of 0.05-20% by weight of the total weight of the composition.

The composition of the present invention, including a protein or carbohydrate as specified above, can be used with various vehicles so as to provide deodorant compositions that can be packaged as an aerosol deodorant, stick deodorant, suspension deodorant, roll-on deodorant, lotion, including body lotion and baby lotions, etc., such compositions containing an effective amount of the specified carbohydrate or protein so as to reduce adherence of malodor-causing bacteria to the skin (for example, so as to reduce adherence of malodor-causing bacteria to the skin at axillary regions of the body).

Illustratively, the compositions according to the present invention can include a vehicle for forming a stick deodorant, for example, a stick deodorant for application to the skin at axillary regions of the body, to inhibit adherence of axillary bacteria to the skin. Thus, the carbohydrate or protein as discussed above can be incorporated with a gelling agent and a liquid vehicle, in forming solid gel sticks. Such sticks can contain a metallic soap or a dibenzyl sorbitol as the gelling agent.

Compositions according to the present invention can include a vehicle for a lotion (such as a body or hand lotion or a baby lotion). Thus, compositions, according to the present invention, being a baby lotion, can be gentle enough (including sufficiently non-acidic and non-basic pH) for a baby's skin (avoiding skin irritation) and can avoid the necessity of an antimicrobial agent, yet which can reduce malodor due to bacterial action.

The present invention is, illustratively, appropriate for reducing adherence of diphtheroids (malodor-causing microorganisms) to the skin in axillary regions of a human body. Accordingly, by this aspect of the present invention, a deodorant composition can be provided which avoids body malodor, e.g., axillary malodor, with no need for antimicrobial agents, and with no need for application of compositions with high or low pH to the skin. Moreover, by use of the present invention, a deodorant composition need not incorporate a fragrance.

Thus, according to the present invention there is provided a deodorant composition which can include only the carbohydrate or protein as the deodorant active ingredient, to inhibit adherence of malodor-causing bacteria to the skin (including in axillary regions). Of course, compositions according to the present invention can also include other deodorant active ingredients, such as a fragrance.

According to another aspect of the present invention, an analytical technique is provided for determining whether specific materials, including specific carbohydrates and proteins, reduce (or inhibit) adherence of malodor-causing bacteria to the skin (e.g., to the skin in axillary regions of a body). According to this analytical technique, differentiated skin cells are used, in combination with malodor-causing aerobic and anaerobic bacteria and the material being investigated, to determine whether such material reduces (inhibits) the adherence of malodor-causing bacteria to the skin. The skin cells utilized are human skin cells, which are differentiated by culturing in keratinocyte growth medium supplemented with a divalent metal compound (especially a divalent calcium compound, such as calcium chloride).

The differentiated skin cells develop keratinized surface structures which are of varying molecular weight, ranging from 50,000 to more than 100,000. This proteinaceous surface structure is close to the keratinized structures that appear on cornyfied surface epithelial cells. Essentially, the proteinaceous structures contain protein, glycoprotein and polysaccharide. The differentiated skin cells grow in mounds in tissue culture and skin bacteria tend to stick to them.

An illustrative keratinocyte growth medium is as follows:

| KGM | (Keratinocyte Growth Medium) |
| --- | --- |
| | (Modified MCDB 153 prepared by Clonetics, Co.) |
| | 10 ng/ml Epidermal Growth Factor (EGF) |
| | 5 µg/ml Insulin |
| | 0.5 µg/ml Hydrocortisone |
| | Anti-microbial agents (Penicillin, Streptomycin, and Amphotericin B) |
| | 0.15 mM Calcium ($Ca^{+2}$) |
| | 0.4% Bovine pituitary extract (BPE) |
| | pH 7.4 |
| | Osmolality = 340 ±4 mOsm/kg |

Undifferentiated epithelial cells require 2 mM $Ca^{+2}$ for differentiation into keratinocytes. Illustratively, a 1M solution of calcium chloride, at a ratio of 2µl of 1M calcium chloride per ml of KGM, is used to provide differentiated epithelial cells. The media is changed every three days in order to maintain this level of calcium in the medium. An optimum time period for achieving cell differentiation is 12-16 days. Less than 10 days is rarely used.

An optimal level of, e.g., calcium chloride is necessary for skin cell differentiation. Too large or too small a level will result in inefficient or no differentiation.

The following references describe use of $Ca^{+2}$ for differentiation, and the contents of each of these references are incorporated herein in their entirety:

1) Pillai, S., et al., "Biochemical and Morphological Characterization of Growth and Differentiation of Normal Human Neonatal Keratinocytes in a Serum-Free Medium", in J. Cellular Physiology. 134:229-237 (1988).

2) Shipley, G.D., et al., "Control of Growth and Differentiation in Vitro of Human Keratinocytes Cultured in Serum-Free Medium", in Arch. Dermatol. 23:1541a (1987).

3) Wilke, M.S., et al., "Biologic Mechanism for the Regulation of Normal Human Keratinocyte Proliferation and Differentiation", in Amer. J. Pathology. 131:171-181 (1988).

The skin cells (differentiated skin cells) are blended with the material to be tested for adherence inhibition, with the bacteria then being added, and thereafter the number of bacteria adhering to the cells are counted in order to determine whether the tested material reduces adherence of malodor-causing bacteria. The bacteria tested can be, e.g., isolated from the human axillary regions.

The skin cells utilized according to the present analytical technique are sterile cells, and due to the differentiation are very similar to surface skin cells of the skin stratum corneum, so that a determination (that adherence of the bacteria to the differentiated skin cells is inhibited) according to the present analytical technique, is a fair test as to whether the material, as part of a deodorant composition, will reduce adherence of malodor-causing bacteria on the skin, such as in axillary regions of the body.

Accordingly, the present invention in all of its aspects, provides compositions, particularly specific deodorant compositions, including specific carbohydrates other than mannose and glucose and derivatives thereof, and/or including specific proteins (glycoproteins), which compositions can be utilized for reducing adherence of malodor-causing bacteria to the skin surface of a body, including in axillary regions of the body, while having sufficient gentleness so as to be incorporated in body lotions such as baby lotions; and provides an analytical technique which permits materials to be investigated, on skin corresponding to surface skin of a human body, as to whether the materials reduce adherence of malodor-causing bacteria to the skin surface.

Thus, compositions can be selected, and used, to avoid body malodor, without such compositions containing antimicrobial agents and without such compositions having a high or low pH. Moreover, deodorant compositions can be provided without the need for incorporating fragrances.

## DETAILED DESCRIPTION OF THE INVENTION

While the invention will be described in connection with specific and preferred embodiments, it will be understood that it is not intended to limit the invention to those embodiments. On the contrary, it is intended to cover all alterations, modifications and equivalents as may be included within the spirit and scope of the invention as defined by the appended claims.

The present invention contemplates compositions, and methods of applying such composition, whereby adhesion of malodor-causing bacteria to the skin of a body (e.g., of a human), including axillary regions of the body, can be inhibited, so as to thereby decrease malodor. The compositions can include various vehicles, whereby various different types of deodorant compositions are provided. For example, depending on the vehicle utilized, a deodorant stick, roll-on, suspension, lotion or aerosol composition can be provided. In each of these compositions, the important component according to the present invention is a carbohydrate, a mixture of different carbohydrates, a protein, or combinations thereof which can inhibit adherence of the malodor-causing bacteria to the skin, so that deodorancy is achieved without the need for antimicrobial agents.

Compositions according to the present invention, containing the carbohydrate or mixture thereof, protein or combination thereof, can be used as a baby lotion, when incorporating a baby lotion vehicle, and is sufficiently gentle to avoid skin irritation of the baby. Thus, a deodorizing baby lotion is provided which reduces malodor and does not include an antimicrobial agent.

Furthermore, the present invention also contemplates an analytical technique whereby various carbohydrates and/or proteins can be tested to determine whether such carbohydrates and/or proteins can inhibit adherence of malodor-causing bacteria to the skin of a body. Accordingly, by this aspect of the present invention various different carbohydrates and/or proteins can be tested, without application to a person, to facilitate determination of whether specific carbohydrates and/or proteins can be utilized to reduce populations of malodor-producing bacteria on a body, and accordingly reduce body malodor.

Prior to further description of the present invention, a short description concerning adherence of bacteria to a body will be provided. Bacteria adhere to and colonize almost any surface. Adherent colonies of bacteria may be observed on and in particles of sand, soil and on various marine, animal and human surfaces; and in and on plant tissue and surfaces. The skin and mucosal surfaces of the gastrointestinal tract, vaginal canal, and oral cavity in man are heavily colonized by a variety of indigenous adherent bacteria. It is recognized that adherence of bacteria to surfaces in man is an important determinant, in colonization of specific sites, as an early event in the pathenogenesis of bacterial infections and the development of body malodor, particularly in the underarm, foot and uro-genital areas.

The mechanism by which bacteria interact with surfaces appears to involve specific molecular ligands or adhesins on the surface of bacteria that interlock by a "lock and key" mechanism with receptor molecules on the surface to be colonized. Such ligands or adhesins may be complex sugar- or oligosaccharide-binding protein structures called lectins, or complex, multi-branched oligosaccharide side chains that bind to complementary sites on the host cell. Molecules which bind to either ligand (adhesin) sites or receptor sites can inhibit bacterial attachment to host surfaces.

According to the present invention, various materials, other than mannose and glucose and oligomers thereof, which can inhibit bacterial attachment to host surfaces (specifically, to the skin surface of, e.g., a human body, including human babies), have been found. Such materials include various carbohydrates, including monosaccharides, oligosaccharides, and polysaccharides, and proteins (including various glycoproteins), as discussed specifically above.

Of the previously listed glycoproteins, laminin and collagen have been reported to bind certain Staphylococci (such as Staphylococci aureus) found on human skin.

Glycoproteins such as fibronectin, laminin, collagen, and α-acid glycoprotein of bovine origin, that can inhibit adherence of malodor-causing bacteria to the skin, from tissue origin or in circulating form in plasma and platelets, are of particular interest. Fibronectin is a 440,000 dalton glycoprotein. An illustrative collagen glycoprotein is a Type III, acid soluble collagen from calf skin, provided by Sigma Chemical Co.

Attention is directed to the following references, describing various glycoproteins; the contents of each of these references are incorporated herein by reference, in their entirety:

1. Grinnell, "Cell Attachment and Spreading Factors", pages 267-92, in Guroff (Ed.), Growth and Maturation Factors (John Wiley & Sons, New York);

2. Kleinman, et al., "Roll of Collagenous Matrices in the Adhesion and Growth of Cells", in J. Cell Biol., 88, 473-485 (1981);

3. Yamada, "Cell Surface Interactions with Extracellular Materials", Annu. Rev. Biochem., 52, 761-799 (1983);

4. Schaeffer, "Bacterial Adherence", Monographs in Urology, pages 131-147 (1984); and

5. Ohtomo, et al., "Adhesion of Staphylococcus Aureus to Fibrinogen, Collagen and Lectin in Relation to Cell Surface Structure", Zbl. Bakt. Hyg., A268, 325-340 (1988).

The carbohydrates listed herein range from simple sugars, such as galactose, to oligomers and polymer carbohydrates (large polysaccharides). Open-chain sugars can be used.

Emulsans, which can form part of the composition of the present invention, are described in the previously mentioned U.S. Patent No. 4,737,359 to Eigen, et al., the contents of which are incorporated herein by reference in their entirety. Such emulsans, as well as the xanthum poms, alginic acids and alginates, guar gums, locust bean poms, gellan poms, carageenans, and fragments thereof, are products produced by various microorganisms. The emulsans and/or other poms and materials referenced herewith which can be utilized according to the present invention are not limited to polysaccharides produced by a specific microorganism, but include xanthum poms or alginic acids and alginates, guar gums, locust bean gums, gellan gums and carageenans produced by different microorganisms.

As indicated previously, compositions, having incorporated therein carbohydrates and/or proteins, according to the present invention, also include vehicles such that the compositions can be used as a stick deodorant, a roll-on, an aerosol, a suspension, a lotion, etc. Illustrative stick deodorant compositions, with amounts of each component, are set forth in the following Formulations A and B. In the Formulations, the percentages are percentages by weight. Of course, the following formulations are merely illustrative, and are not limiting of the present invention.

Formulation A

| Propylene Glycol | 20 - 80% |
|---|---|
| Water | 0 - 50% |
| Subject Carbohydrate | 0.05 - 20% |
| EDTA | 0 - 5% |
| Metallic Soap (such as sodium stearate) | 3 - 10% |
| Fragrance | 0 - 3% |

Formulation B

| Dibenzyl Monosorbitol Acetal or Other Dibenzyl Sorbitols | 2 - 10% |
|---|---|
| Propylene Glycol | 20 - 80% |
| Water | 0 - 50% |
| Subject Carbohydrate | 0.05 - 20% |
| Ethyl Alcohol | 0 - 40% |
| Other Glycols | 0 - 10% |
| Oils, Silicones, Methylated Spirits | 0 - 20% |
| Other Gellants Such as Hydroxy Ethyl Cellulose | 0 - 2% |

The compositions of the present invention can be formed by conventional techniques, for example, conventional mixing techniques, and can include other components, apart from the vehicle and adherence-inhibiting material, that are conventional in the art. As for various materials utilized in conventional deodorant compositions, e.g., for application to axial regions, note, for example, U.S. Patent No. 4,440,742 to Marschner (particularly with respect to sticks), and U.S. Patent No. 4,152,416 to Spitzer, et al. (particularly with respect to aerosols), the contents of each of which are incorporated herein by reference in their entirety.

The compositions according to the present invention can be conventionally incorporated in a package, and used, as done conventionally.

While the following examples are illustrative of the invention, it will be understood that it is not intended to limit the invention to these examples.

EXAMPLES 1 - 10 (SOAP-BASED DEODORANT STICKS) are set out in Tables 1A and 1B.

Table 1A

| Ingredient | (% w/w) | | | | |
|---|---|---|---|---|---|
| | 1 | 2 | 3 | 4 | 5 |
| Propylene Glycol | 65.75 | 65.0 | 56.0 | 68.0 | 62.0 |
| Water | 20.0 | 20.0 | 20.0 | 20.0 | 20.0 |
| D-Galactose | 2.5 | 5.0 | ---- | ---- | 6.0 |
| N-Acetylglucosamine | 2.5 | ---- | 15.0 | 2.5 | 2.0 |
| N-Acetylgalactosamine | ---- | ---- | ---- | 2.5 | 2.0 |
| D-Fucose | ---- | ---- | ---- | ---- | 1.0 |
| Fibronectin | ---- | ---- | ---- | ---- | ---- |
| Xanthan Gum | ---- | ---- | ---- | ---- | ---- |
| Sodium Alginate | ---- | ---- | ---- | ---- | ---- |
| Fragrance | ---- | 1.0 | ---- | ---- | ---- |
| Na$_4$EDTA | 2.0 | 2.0 | 2.0 | ---- | ---- |
| Irgasan DP-300 | 0.25 | ---- | ---- | ---- | ---- |
| Sodium Stearate | 7.0 | 7.0 | 7.0 | 7.0 | 7.0 |

Table 1B

| Ingredient | (% w/w) | | | | |
|---|---|---|---|---|---|
| | 6 | 7 | 8 | 9 | 10 |
| Propylene Glycol | 53.0 | 72.0 | 70.0 | 70.0 | 70.0 |
| Water | 20.0 | 21.0 | 20.0 | 21.0 | 20.0 |
| D-Galactose | 5.0 | ---- | 0.5 | ---- | 0.5 |
| N-Acetylglucosamine | ---- | ---- | ---- | ---- | ----- |
| N-Acetylgalactosamine | ---- | ---- | 0.5 | ---- | ---- |
| D-Fucose | 15.0 | ---- | ---- | ---- | ---- |
| Fibronectin | ---- | 1.0 | ---- | 1.0 | ---- |
| Xanthan Gum | ---- | 0.5 | ---- | ---- | ---- |
| Sodium Alginate | ---- | ---- | 1.0 | ---- | 1.5 |
| Fragrance | ---- | ---- | 1.0 | ---- | 1.0 |
| $Na_4EDTA$ | ---- | ---- | ---- | ---- | 2.0 |
| Irgasan DP-300 | ---- | ---- | ---- | ---- | ---- |
| Sodium Stearate | 7.0 | 5.5 | 7.0 | 7.0 | 5.0 |

EXAMPLES 11 - 16 (DBMSA-BASED DEODORANT STICKS) are set out in Tables 2A and 2B

Table 2A

| Ingredient | (% w/w) | | |
|---|---|---|---|
| | 11 | 12 | 13 |
| Dibenzyl Monosorbitol Acetal | 2.5 | 1.5 | 3.0 |
| Propylene Glycol | 55.0 | 66.0 | ---- |
| Dipropylene Glycol | ---- | ---- | 37.0 |
| Water | 24.5 | 24.5 | 10.0 |
| Ethyl Alcohol | ---- | ---- | 30.0 |
| Methyl Pyrrolidone | 5.0 | ---- | ---- |
| Acetamide MEA | ---- | 5.0 | 7.0 |
| Finesolve (Alcohol Benzoate) | 5.0 | 2.0 | 5.0 |
| Klucel MF | 0.5 | ---- | ---- |
| D-Galactose | 2.5 | ---- | 7.0 |
| N-Acetylglucosamine | 2.5 | ---- | ---- |
| Xanthan Gum | ---- | 1.0 | ---- |
| Fibronectin | ---- | ---- | ---- |
| Na$_4$EDTA | 1.0 | ---- | 1.0 |
| Fragrance | 1.0 | ---- | ---- |

Table 2B

| Ingredient | (% w/w) | | |
|---|---|---|---|
| | 14 | 15 | 16 |
| Dibenzyl Monosorbitol Acetal | 1.5 | 1.5 | 1.5 |
| Propylene Glycol | 66.0 | 66.0 | 66.5 |
| Dipropylene Glycol | ---- | ---- | ---- |
| Water | 20.0 | 22.2 | 20.0 |
| Ethyl Alcohol | ---- | ---- | ---- |
| Methyl Pyrrolidone | ---- | 5.0 | 5.0 |
| Acetamide MEA | 5.0 | ---- | ---- |
| Finesolve (Alcohol Benzoate) | 2.0 | 2.5 | 2.0 |
| Klucel MF | 0.5 | ---- | ---- |
| D-Galactose | ---- | ---- | 2.0 |
| N-Acetylglucosamine | 5.0 | ---- | 2.0 |
| Xanthan Gum | ---- | ---- | 0.5 |
| Fibronectin | ---- | 0.8 | 0.5 |
| Na$_4$EDTA | ---- | 1.0 | ---- |
| Fragrance | ---- | 1.0 | ---- |

**EP 0 561 489 A2**

EXAMPLES 17 - 21 (SUSPENSION ROLL-ONS) are set out in Table 3

Table 3

| Ingredient | (% w/w) | | | | |
|---|---|---|---|---|---|
| | 17 | 18 | 19 | 20 | 21 |
| Talc | 15.0 | 15.0 | 18.0 | 18.0 | 18.0 |
| Bentone 38 | 3.0 | 2.5 | 3.0 | 3.0 | 3.5 |
| SD-40 Alcohol | 1.5 | 1.2 | 1.5 | 1.5 | 1.5 |
| Cyclomethicone | 73.5 | 67.8 | 73.5 | 76.3 | 75.0 |
| Fragrance | 1.0 | ---- | 1.0 | ---- | ---- |
| $Na_4EDTA$ | 1.0 | ---- | ---- | 1.0 | 1.0 |
| D-Galactose | 3.0 | 3.0 | 1.0 | ---- | ---- |
| N-Acetylglucosamine | 1.0 | 10.0 | 1.0 | ---- | ---- |
| Sodium Alginate | 1.0 | 0.5 | 0.5 | ---- | 0.5 |
| Xanthan Gum | ---- | ---- | 0.5 | ---- | 0.3 |
| Fibronectin | ---- | ---- | ---- | 0.2 | 0.2 |

Examples 22 and 23

As stated in the foregoing, materials inhibiting adherence of malodor-causing bacteria can be incorporated in baby lotions. Illustrative baby lotions containing such anti-adherence agents are shown in the following Tables 4 (Example 22) and 5 (Example 23).

14

## Table 4
### BABY LOTION WITH ANTI-ADHERENCE AGENT

|  | With Quaternary Compound |
|---|---|
| Water | 85.60 |
| Glycerine | 6.07 |
| Lapyrium chloride | 0.10 |
| Benzalkonium chloride | 0.10 |
| Red Color sol'n | 0.17 |
| Violet Color sol'n | 0.06 |
| Methyl Paraben | 0.10 |
| Lanolin Base | 1.24 |
| Lanolin | 0.13 |
| Glyceryl Stearate | 1.92 |
| Cetyl Alcohol | 1.92 |
| Glyceryl Stearate & PEG 100 Stearate | 1.25 |
| Propyl Paraben | 0.04 |
| P.G. Alginate[1] | 0.81 |
| Sodium Alginate | 0.20 |
| Dow Corning 544 Fluid | 0.01 |
| Fragrance | 0.30 |
|  | 100.00 |

(1)   P.G. Alginate is propylene glycol alginate.

## Table 5

### BABY LOTION WITH ANTI-ADHERENCE AGENT

|  | Without Quaternary Compound |
| --- | --- |
| Water | 85.27 |
| Glycerine | 6.04 |
| Lapyrium chloride | -- |
| Benzalkonium chloride | 0.10 |
| Red Color sol'n | 0.17 |
| Violet Color sol'n | 0.06 |
| Methyl Paraben | 0.10 |
| Lanolin Base | 1.33 |
| Lanolin | 0.13 |
| Glyceryl Stearate | 2.05 |
| Cetyl Alcohol | 2.05 |
| Glyceryl Stearate & PEG 100 Stearate | 1.34 |
| Propyl Paraben | 0.05 |
| P.G. Alginate[1] | 0.80 |
| Sodium Alginate | 0.20 |
| Dow Corning 544 Fluid | 0.01 |
| Fragrance | 0.30 |
|  | 100.00 |

(1)   P.G. Alginate is propylene glycol alginate.

Efficacy of deodorant compositions according to the present invention is demonstrated in the following.

Six (6) male volunteers abstained from the use of underarm antiperspirant products for a period of two weeks. Volunteers used only standardized deodorant product. Two days prior to the first application of test material, volunteers abstained from the use of any underarm product; volunteers did not wash their axillae for one day prior to this first application. The axillae of the six (6) volunteers were evaluated for underarm malodor on the morning of the first test material application to ensure that significant malodor was present. After a one-minute washing with tepid water, 0.5 grams test material was applied to the axillae. The test material of Example 1, formulated as a stick, was applied to one axilla, while the deodorant stick base (without carbohydrate or protein) was applied to the contralateral axilla. Odor evaluations were made by the subjects 12 hours and 24 hours following product application by inserting and rotating a glass test tube in the axilla and evaluating the odor on the test tube. The odor present on the test tube was graded on a scale of 0-10, 0 being no noticeable odor and 10 being a strong repugnant odor.

The results of the test demonstrate that the test material from Example 1 produced a greater reduction in axillary malodor than a similar formula, without the carbohydrates. The Table below provides the numerical test results:

EP 0 561 489 A2

Table 6

| Average Odor Score | | |
|---|---|---|
| | Hours Post Application | |
| | 12 | 14 |
| Product | | |
| Test Material | 2.7 | 3.0 |
| Control | 4.5 | 3.7 |

The following Table 7 shows efficacy of the above baby lotions containing an anti-adherence agent according to the present invention in inhibiting adherence of bacteria to the skin.

Table 7

| In-Vitro Bacteria Adherance Data | | | |
|---|---|---|---|
| | Bacteria/20 Cells | | % |
| No Treatment | Run 1 | Run 2 | Inhibition |
| Corynebacterium Group F-1 | 175 | 202 | |
| Corynebacterium Group C | 233 | 258 | |
| Corynebacterium Pseudotuberculosis | 222 | 272 | |
| Corynebacterium Xerosis | 180 | 208 | |
| Corynebacterium | 500 | 500 | |
| Baby Lotion with Anti-Adherence Agent | | | |
| Corynebacterium Group F-1 | 28 | 43 | 81 |
| Corynebacterium Group C | 46 | 37 | 83 |
| Corynebacterium Pseudotuberculosis | 29 | 25 | 89 |
| Corynebacterium Xerosis | 52 | 33 | 78 |
| Corynebacterium | 78 | 57 | 87 |

A specific example of the analytical technique, discussed previously, for determining whether a specific material inhibits adherence of malodor-causing bacteria to the skin, particularly the skin at axillary regions of a body, will be set forth in the following. As will be seen, adherence-inhibiting effect can be investigated without application to a living body.

The results shown in connection with the following Analytical Example set forth effectiveness, as an adherence-inhibiting material, of various carbohydrates and proteins within the scope of the present invention, and also set forth comparisons with other materials outside the scope of the present invention. Of course, this example is not limiting, and is merely illustrative of the present invention.

Analytical Example

Microorganisms.

Skin microorganisms were isolated from the axillae of ten healthy human volunteers (male and female). Corynebacterium diphtheriae, Corynebacterium group J-K, and Escherichia coli 1677 were also obtained. The

17

latter bacteria were included as controls for the adherence experiments. Microorganisms were maintained either by subculture on blood agar plates or trypticase soy agar base with 5% defibrinated sheep's blood. Stock cultures were frozen at -20°C in semi-solid trypticase soy agar for aerobes and chopped meat carbohydrate broth for anaerobes. For in vitro adherence assays the lipophilic corynebacteria were serially subcultured three times for periods of 24 hrs. in tryptose broth with 0.5% Tween-80.

## Isolation and Identification

Isolation of microorganisms from the axillae was performed by addition of 1 ml of a 0.1% Triton X-100 solution onto a sterile glass cylinder placed firmly on the skin in the axilla (total area 3.8cm$^2$). The fluid was stirred vigorously for 1 min. with a Teflon rod and collected. The latter procedure was repeated and the two samples were pooled. To help in the recovery of anaerobes, oxygen-free $CO_2$ was applied over the collected samples and the tubes were capped and cooled to 4°C. All samples were processed within 8 hrs. of collection. Volunteers were asked not to use deodorants and only to wash with a mild soap for 8 days prior to sampling their axillae. Individual samples were serially diluted and plated onto non-prereduced and prereduced blood agar plates (BAP). Aerobic and anaerobic incubation conditions were maintained for 3-5 days at 37°C, at which time final bacterial counts of both aerobes and anaerobes were made. Axillary samples were processed inside an anaerobic glove box. All microorganisms were tested for lipophilicity by subculture on BAP with 0.5% Tween-80 and incubation under aerobic or anaerobic conditions at 37°C for 48 hrs. Microorganisms that demonstrated enhanced growth or pigmentation on this lipid supplemented growth medium were considered lipophilic.

## Epithelial Cells

Human epithelial (HE) cells (primary culture from normal human epidermal keratinocytes) were used to assess the adherence of the axillary microorganisms. Cells were resuspended in phosphate-buffered saline (PBS) and washed three times by differential centrifugation (500 rpm) with a vigorous agitation between washes (19). Differentiation of HE cells was induced by addition of calcium chloride ($CaCl_2$) to a concentration of 2 mM/L. Differentiation was assessed by mound formation (3 to 5 layers of cells) during tissue culture and by a modified Ayoub-Shklar's stain for differentiated epithelial cells. Human epithelial cells were grown in keratinocyte growth medium. Cell lines were cultured under an atmosphere of 5% $CO_2$ at 37°C. Cell lines were subcultured at the following intervals: 8 days for undifferentiated HE cells, and 8-16 days for differentiated HE cells. Both cell lines were harvested by addition of a trypsin (0.25 g/L) and EDTA (0.1 g/L) solution. HE cells were washed with Hepes buffer prior to the trypsinization and an antitrypsin solution was added to neutralize the enzymatic reaction. Cells were allowed to recover from the trypsinization procedure for 6-8 hrs. in growth medium under a 5% $CO_2$ atmosphere at 37°C. Harvested cells were washed three times by centrifugation (3 min., 1500 rpm) with PBS (pH 7.2, 0.067 M) and resuspended in Dulbecco's phosphate-buffered saline to a final concentration of
0.5-1.0 x 10$^6$ cells/ml. Suspensions were standardized in a hemacytometer prior to use.

## In Vitro Bacterial Adherence Assays

Fifty $\mu$l (microlitres) of the individual bacterial suspensions (1.5 x 10$^8$ bacteria/ml for anaerobes and 3.0 x 10$^8$ bacteria/ml for aerobes) were added to 50 $\mu$l (microlitres) of cell suspension (0.5 to 1.0 x 10$^6$ cells/ml) and incubated for 30 min. while rocking at room temperature. A 25 $\mu$l (microlitres) inoculum was vacuum filtered onto a polycarbonate filter (pore size 8.0 $\mu$m (micrometres)). The cells on the filter were washed three times with sterile distilled water to remove non-adherent bacteria. After fixation with 95% ethanol, the cells with attached bacteria were Gram stained on the filter. The number of bacteria attaching to 20 epithelial cells was counted using a light microscope with a tool magnification of 1000x. Experiments were run in duplicate and three individual blind readings were made on each sample.

## Inhibition of Bacterial Adherence

The following compounds were assayed for their effects on the adherence of skin microorganisms to epithelial cells: 50 $\mu$g/ml (micrograms/ml) human fibronectin, wheat germ agglutin (Triticum Vulgaris) and jack bean lectin (concanavalin A), 17 mg/ml final concentrations of D(+) fucose, D(+) galactose, D(+) mannose and N-acetyl-D-glucosamine (NAGA), sucrose, emulsan, and dextran, and 3.4 mg/ml hydrolyzed sodium alginate. In the adherence inhibition assays 50 $\mu$l (microlitres) of the compound to be tested was added to the HE cells in vitro for 5 min. before adding the bacterial suspensions.

Statistical Analysis

The Student's T-test for determining significant differences between pairs of data was used to compare the results from the adherence and inhibition of adherence assays. A Minitab program for IBM computers was employed and the p value was set at 0.05 before the experiments were run.

Results

In connection with these results, note Tables 8-11, which are set forth after this discussion of the results.

Forty-nine aerobic and anaerobic microorganisms were isolated from the axillae of 10 healthy male and female human volunteers (Table 8). As a group, the staphyloccoci were most frequently isolated and S. hominis was isolated from 8 of the 10 volunteers. Lipophilic aerobes and anaerobes were isolated from 7 of 10 subjects (Table 3). The number of bacteria isolated varied widely from $5.0 \times 10^2$ to $2.9 \times 10^6$ CFU/ml/2.8 cm$^2$ for lipophilic aerobes and from $2.0 \times 10^2$ to $5.0 \times 10^6$ CFU/ml/2.8 cm$^2$ for lipophilic anaerobes. Non-lipophilic aerobes predominated in all of the subjects and a wide concentration range ($4.0 \times 10^1$ to $6.9 \times 10^7$ CFU/ml/2.8 cm$^2$) was evident (Table 8).

Nine aerobic and several anaerobic diphtheroids were tested for their adherence to undifferentiated and differentiated HE cells. In addition to these microorganisms, a Corynebacterium group J-K, Corynebacterium diphtheriae, and Escherichia coli (strain 1677) were included as controls. The average number (based on 6 readings of 2 assays) of aerobic diphtheroids adhering to 20 epithelial cells is shown in Table 9. In general, both aerobic (Table 9) and anaerobic diphtheroids adhered better to differentiated HE cells than to undifferentiated HE cells (p <0.05).

Mannose, galactose, fucose, NAGA and FN were assayed for their capacity to interfere with the adherence of aerobic diphtheroids to undifferentiated and differentiated HE cells (Table 10). All of the compounds had the capacity to inhibit attachment of skin bacteria to differentiated or undifferentiated HE cells. Overall, mannose appeared to have the best capacity to inhibit bacterial adherence to undifferentiated cells (87.5 to 99.4% inhibition); however, mannose inhibition of adherence to differentiated cells (53.9 to 90.3%) varied with the microorganism tested and was not consistently high with all of the isolates tested.

With the exception of a poor capacity to inhibit attachment of C. pseudo-tuberculosis (isolate 1) and Corynebacterium (group J-K), galactose was able to inhibit attachment of aerobic skin bacteria to differentiated epithelial cells better (70 to 90% inhibition; Table 10) than mannose, NAGA, fucose and FN. In general, fucose, NAGA and FN were consistently able to inhibit attachment of aerobic skin bacteria to both differentiated and undifferentiated HE cells; however, inhibition of adherence was more evident with differentiated HE cells than with undifferentiated cells.

Sucrose, emulsan, dextran, hydrolyzed sodium alginate, jack bean lectin and wheat germ lectin were assayed for their capacity to interfere with the adherence of aerobic diphtheroids to differentiated HE cells (Table 11). Emulsan and hydrolyzed sodium alginate appeared to have the best capacity to inhibit bacterial adherence to differentiated cells (emulsan 76.8% to 96.8% and sodium alginate 73.9% to 93.7%). Sucrose and dextran demonstrated a poor capacity to inhibit adherence (-12.8% to 22.6% and -9.0% to 53.0%, respectively). The two lectins promoted bacterial adherence to all differentiated cells. The increase in adherence was so large that there were too many bacteria to count; therefore, no data for these lectins are provided in Table 11.

As seen in the foregoing and in Tables 8-10, aerobic and anaerobic diphtheroids demonstrated an increased capacity to adhere to differentiated HE cells. These results are due to the fact that, of the cells used, the differentiated HE cells most closely resemble the surface epithelial cells found in the epidermis. Variability in the adherence capacity of different isolates of aerobic and anaerobic diphtheroids was evident. For example, C. minutissium (isolate 2), Corynebacterium group G-2 and P. acnes serotype 1 (isolate 3) demonstrated a greater capacity to adhere to differentiated HE cells than the other aerobic and anaerobic isolates.

Although the adherence inhibition data provided herewithin refers to aerobic bacteria, this analytical method equally applies to anaerobic bacteria.

Although the adherence of different bacterial isolates was inhibited to varying degrees by the compounds tested, mannose gave the best inhibition of bacterial adherence to undifferentiated HE cells, whereas galactose, FN, NAGA, emulsan and hydrolyzed sodium alginate showed a good capacity to inhibit adherence to differentiated HE cells. These adherence studies indicate that a mannose binding adhesin on the cell wall of diphtheroids may be implicated in their adherence to undifferentiated HE cells. Similarly, mannose and galactose, NAGA and other receptors may be involved in bacterial adherence to differentiated HE cells. Fibronectin may also play a role in attachment of diphtheroids to undifferentiated and differentiated HE cells. These results indicate that several factors mediate the adherence of these skin microorganisms to epithelial cells. The in vitro adherence and adherence inhibition assays described herein demonstrate that bacterial adhesins and receptors on epithelial cells may play an important role in skin ecology, and may explain the predominance and persistence of certain groups of bacteria in these ecosystems.

As seen in the foregoing Analytical Example, the use of epithelial cells which are derived from human skin provides a convenient in vitro model for the study of adherence of skin microorganisms. There have been several attempts to perform bacterial adherence studies directly on human skin; however, the methodology substantially modifies the skin environment and thus its indigenous microflora. In addition, other normal flora microorganisms may interfere with such "in situ" studies, and certain skin cleansing agents could modify adhesins and receptors.

TABLE 8. Bacteria Isolated from Human Axillae

| Bacterial Strains | Frequency of Isolation from 10 Subjects | Range Observed (CFU/ml/2.8cm$^2$) |
|---|---|---|
| AEROBIC, NON-LIPOPHILIC: | | |
| Staphylococci | | |
| S. hominis | 8/10 | $1.9 \times 10^3$ -$5.0 \times 10^6$ |
| S.sciuri | 1/10 | $1.7 \times 10^4$ |
| S. haemolyticus | 5/10 | $1.2 \times 10^2$ -$9.0 \times 10^5$ |
| S. warneri | 3/10 | $1.7 \times 10^4$ -$6.9 \times 10^7$ |
| S. epidermidis | 5/10 | $3.2 \times 10^2$ -$3.6 \times 10^4$ |
| S. caprae | 1/10 | $8.5 \times 10^5$ |
| S. auricularis | 1/10 | $1.6 \times 10^5$ |
| S. capitas | 1/10 | $1.3 \times 10^7$ |
| Micrococci | | |
| M. luteus | 3/10 | $8.0 \times 10^1$ -$5.3 \times 10^6$ |
| M. sedentarius | 1/10 | $6.4 \times 10^3$ |
| M. varians | 1/10 | $4.0 \times 10^1$ |
| Flavobacterium odorans | 1/10 | $1.5 \times 10^5$ |
| AEROBIC, LIPOPHILIC: | | |
| Corynebacteria | | |
| C. minutissium | 2/10 | $2.4 \times 10^3$ -$1.8 \times 10^4$ |
| C. pseudotuberculosis | 1/10 | $2.9 \times 10^6$ |
| C. group F-1 | 1/10 | $6.0 \times 10^2$ |
| C. group J-K | 1/10 | $1.7 \times 10^6$ |
| C. group G-2 | 1/10 | $8.0 \times 10^4$ |
| C. xerosis | 1/10 | $5.0 \times 10^2$ |
| C. group C | 1/10 | $4.4 \times 10^3$ |
| ANAEROBIC, LIPOPHILIC: | | |

| | | |
|---|---|---|
| Propionibacterium acnes serotype 1 | 3/10 | $7.0 \times 10^2 - 1.3 \times 10^5$ |
| Propionibacterium acnes serotype 2 | 4/10 | $2.0 \times 10^2 - 5.0 \times 10^6$ |
| Propionibacterium thoenii | 1/10 | $3.3 \times 10^4$ |
| Lactobacillus sp. | 1/10 | $4.4 \times 10^6$ |
| Streptococcus intermedius | 1/10 | $1.6 \times 10^4$ |

**TABLE 9. Adherence of Aerobic Skin Bacteria to Human Epithelial Cells**

| Bacterial Strains | Human Epithelial (HE) Cells[c] Undifferentiated | Differentiated |
|---|---|---|
| C. minutissium | | |
| Isolate 1 | $193 \pm 11$[b] | $190 \pm 5$ |
| Isolate 2 | $128 \pm 12$ | $498 \pm 11$ |
| C. pseudotuberculosis | | |
| Isolate 1 | $172 \pm 7$ | $234 \pm 9$ |
| Isolate 2 | $158 \pm 9$ | $210 \pm 14$ |
| C. group F-1 | $183 \pm 10$ | $434 \pm 22$ |
| C. group G-2 | $253 \pm 26$ | >500 |
| C. xerosis | $201 \pm 15$ | $266 \pm 16$ |
| C. group C | $169 \pm 7$ | $324 \pm 6$ |
| C. group J-K | $191 \pm 7$ | $316 \pm 28$ |
| C. group J-K (SLH) | $182 \pm 11$ | $201 \pm 24$ |
| C. diphtheriae (SLH) | $94 \pm 9$ | $115 \pm 6$ |
| E. coli 1677[d] | $271 \pm 16$ | $267 \pm 14$ |

[a]    Average cell sizes: HE undifferentiated - 12.2 microns and HE differentiated - 14.3 microns.

[b]    Average number of bacteria attaching to 20 epithelial cells $\pm$ SE.

[c]    Differentiation of human epithelial (HE) cells was induced by addition of calcium chloride (at a final concentration of 2 mmol/L to the growth medium.

[d]    E. coli used as a gram-negative control for adherence studies.

**TABLE 10A.   Inhibition of Adherence of Aerobic Bacteria to Human Epithelial (HE) Cells.**

| Bacterial Strain | HE Cells | Man-nose | Galac-tose | Fucose |
|---|---|---|---|---|
| *C. pseudotuberculosis* Isolate 1 | Undifferentiated Differentiated[c] | 94.2[a] 53.9 | -4.6 15.8 | -8.7 35.5 |
| *C.* group F-1 | Undifferentiated Differentiated | 95.6 78.3 | 26.8 88.3 | 27.3 89.2 |
| *C.* group J-K | Undifferentiated Differentiated | 92.2 69.3 | 8.4 77.2 | 25.1 54.4 |
| *C. minutissium* Isolate 2 | Undifferentiated Differentiated | 87.5 90.3 | 24.2 95.8 | 57.0 84.5 |
| *C.* group G-2 | Undifferentiated Differentiated | 88.5 78.0 | 60.5 84.4 | 44.3 77.8 |
| *C. xerosis* | Undifferentiated Differentiated | 96.5 88.4 | 60.7 73.3 | 79.6 77.8 |
| *C.* group C | Undifferentiated Differentiated | 99.4 71.0 | 78.7 85.8 | 49.7 63.0 |

[a]   Percentage inhibition of bacterial attachment by 50 $\mu$l of a 5% (17 mg/ml) carbohydrate.

[b]   Percentage inhibition of bacterial attachment by 50 $\mu$g/ml solution of fibronectin.

[c]   Differentiation of human epithelial cells induced by addition of calcium chloride to a final concentration of 2 mmol/L.

**TABLE 10B. Inhibition of Adherence of Aerobic Bacteria to Human Epithelial (HE) Cells.**

| Bacterial Strain | HE Cells | NAGA | Fibro-nectin |
|---|---|---|---|
| C. pseudotuberculosis Isolate 1 | Undifferentiated Differentiated[c] | 43.6 76.1 | 63.4[b] 63.7 |
| C. group F-1 | Undifferentiated Differentiated | 59.0 86.9 | 45.9 83.9 |
| C. group J-K | Undifferentiated Differentiated | 53.9 81.7 | 34.6 88.9 |
| C. minutissium Isolate 2 | Undifferentiated Differentiated | 65.6 88.6 | 65.6 95.0 |
| C. group G-2 | Undifferentiated Differentiated | 59.3 87.8 | 53.4 73.2 |
| C. xerosis | Undifferentiated Differentiated | 73.1 82.0 | 63.2 72.9 |
| C. group C | Undifferentiated Differentiated | 62.1 80.6 | 36.4 76.9 |

[a] Percentage inhibition of bacterial attachment by 50 $\mu$l of a 5% (17 mg/ml) carbohydrate.

[b] Percentage inhibition of bacterial attachment by 50 $\mu$g/ml solution of fibronectin.

[c] Differentiation of human epithelial cells induced by addition of calcium chloride to a final concentration of 2 mmol/L.

## TABLE 11. Inhibition of Adherence of Aerobic Bacteria to Differentiate Human Epithelial (HE) Cells

| Bacterial Strain | Sucrose[a] | Dextran[a] | Emulsan[a] | Hydrolyzed[b] Na Alginate |
|---|---|---|---|---|
| C. pseudotuberculosis Isolate 1 | 22.6 | 53.0 | 76.8 | 82.6 |
| C. group F-1 | 11.2 | -9.0 | 96.8 | - |
| C. group J-K | 13.9 | - | 95.5 | 73.9 |
| C. minutissium Isolate 2 | -12.8 | 46.0 | 90.1 | 89.0 |
| C. group G-2 | - | - | - | - |
| C. xerosis | 8.4 | 38.0 | 94.1 | 89.8 |
| C. group C | 10.0 | - | 69.2 | 93.7 |

[a]    Percentage inhibition of bacterial attachment by 50 $\mu$l of a 5% (17 g/ml) carbohydrate solution.

[b]    Percentage inhibition of bacterial attachment by 50 $\mu$g/ml of a 1% (3.4 g/ml) carbohydrate solution.

[c]    Differentiation of HE cells induced by addition of calcium chloride to a final concentration of 2 mmol/L.

As seen in the foregoing, use of differentiated cells in the analytical technique provides different results than the results obtained through use of undifferentiated cells. Furthermore, since the differentiated cells most closely resemble the surface epithelial cells found in the epidermis, the present analytical technique provides satisfactory results in determining whether various materials are efficacious in inhibiting adherence of malodor-causing bacteria to the skin, so as to provide efficacious deodorant compositions.

INDUSTRIAL APPLICABILITY

Compositions of the present invention have applicability as deodorants to prevent body malodor, including malodor arising in the axillary regions of the body. The compositions, which need not contain an antimicrobial agent, are sufficiently gentle to the skin to be used, with an appropriate vehicle, as a baby lotion. The compositions of the present invention can be formed by conventional mixing techniques, and can be utilized as done conventionally (for example, depending on the vehicle used in the compositions, the compositions can be in the form of a stick deodorant, incorporated in a stick package, and applied to the skin as done conventionally with stick deodorant).

In addition, the analytical technique of the present invention provides an in vitro model for study of adherence of
skin microorganisms, without necessity of performing techniques in vivos on human skin.

While we have shown and described several embodiments in accordance with the present invention, it is understood that the same is not limited thereto but is susceptible of numerous changes and modifications as known to one having ordinary skill in the art and we therefore do not wish to be limited to the details shown and described herein, but intend to cover all such modifications as are encompassed by the scope of the appended claims.

## Claims

1. A deodorant composition for reducing body malodor, comprising a deodorant active ingredient in a vehicle, the deodorant active ingredient reducing adherence of malodor-causing aerobic or anaerobic bacteria to skin of the body characterised in that the deodorant active ingredient is a glycoprotein that reduces the said adherence; a monosaccharide or a derivative, oligomer or mixture of the said monosaccharides, that reduces the said adherence, the said monosaccharide being other than mannose or glucose or oligomers or mixtures thereof; or a water-soluble polysaccharide or a fragment thereof that reduces the said adherence; the said deodorant active ingredient being present in the composition in an amount sufficient to reduce malodor without the need to incorporate an antimicrobial agent in the deodorant composition.

2. A deodorant composition as claimed in Claim 1 characterised in that it does not contain an antimicrobial agent.

3. A deodorant composition as claimed in Claim 1 or Claim 2, characterised in that the said vehicle is a vehicle for a body or a baby lotion, the deodorant composition being a body lotion or a baby lotion.

4. A deodorant composition as claimed in Claim 1 or Claim 2, characterised in that the said vehicle is a vehicle for a deodorant for axillary regions of a human body, the deodorant composition being an anxillary region deodorant composition.

5. A deodorant composition as claimed in Claim 4, characterised in that the vehicle includes a gelling agent, whereby the composition is an axillary region stick deodorant composition.

6. A deodorant composition as claimed in any one of the preceding claims characterised in that the composition does not contain a fragrance.

7. A deodorant composition as claimed in any one of the preceding claims, characterised in that the ingredient that reduces adherence of malodor-causing bacteria to the skin is included in an amount of 0.05-20% by weight of the weight of the composition.

8. A deodorant composition as claimed in Claim 7 characterised in that the ingredient that reduces adherence of malodor-causing bacteria to the skin is included in an amount of 0.05-1.5% by weight of the weight of the composition.

9. A deodorant composition as claimed in any one of the preceding claims characterised in that the said deodorant active ingredient comprises a glycoprotein.

10. A deodorant composition as claimed in Claim 9, characterised in that the said glycoprotein comprises fibronectin, laminin, collagen, or an a-acid glycoprotein, or a mixture thereof.

11. A deodorant composition as claimed in any one of the preceding claims characterised in that the said ingredient comprises a polysaccharide or a fragment thereof.

12. A deodorant composition as claimed in Claim 11 characterised in that the said deodorant active ingredient is a polysaccharide, and the polysaccharide comprises emulsan, xanthan gum, alginic acid or an alginate thereof, guar gum, locust bean gum, gellan gum, or a carageenan or a mixture thereof.

13. A deodorant composition as claimed in any one of the preceding claims, characterised in that the deodorant active ingredient comprises a monosaccharide or a derivative, oligomer, or mixture of monosaccharides, the monosaccharide being other than mannose or glucose or an oligomer or mixture thereof.

14. A deodorant composition as claimed in Claim 13 characterised in that the said deodorant active ingredient comprises galactose, N-acetyl-D-galactosamine, fucose, N-acetyl-D-glucosamine, D-glucuronic acid, or a derivative or oligomer thereof, or a mixture thereof.

15. A deodorant composition as claimed in any one of Claims 1 to 5 characterised in that the deodorant active ingredient is an ingredient that inhibits adherence of malodor-producing corynebacterium to the body.

16. A deodorant composition as claimed in any one of Claims 1 to 5 characterised in that the ingredient is an ingredient that inhibits adherence of malodor-producing diphtheroids on human skin of the body.

17. A deodorant composition as claimed in any one of the preceding claims characterised in that the deodorant active ingredient is at least partly soluble in the vehicle.

18. A deodorant composition as claimed in Claim 17 characterised in that the vehicle is aqueous and the deodorant active ingredient is at least partly in aqueous solution.

19. An analytical method for determining whether a predetermined material can inhibit adherence of aerobic and anaerobic bacteria to the skin of a body, comprising the steps of:
(a) preparing skin cells so as to provide differentiated cells, by culturing skin cells in a growth medium supplemented with a compound containing a divalent metal;
(b) preparing bacteria;
(c) placing the predetermined material together with the prepared differentiated skin cells;
(d) after step (c), adding the bacteria to the mixture of predetermined material and differentiated skin cells; and
(e) counting the number of bacteria adhering to the differentiated skin cells.

20. A method as claimed in Claim 19 characterised in that it includes the further steps of:
(f) repeating the process with another predetermined material or with a control free of predetermined material; and
(g) comparing the results.

21. An analytical method as claimed in Claim 19 or Claim 20 characterised in that the compound containing a divalent metal contains $Ca^{2+}$.

22. An analytical method as claimed in Claim 21 characterised in that the compound containing a divalent metal is $CaCl_2$.

23. A method of preparing a deodorant composition characterised in that it comprises screening a candidate active ingredient by means of the method claimed in Claim 19, selecting a candidate effective to inhibit adherence of aerobic and/or anaerobic bacteria to the skin of a body and formulating the said selected candidate with a vehicle to provide a deodorant composition.

24. A method as claimed in Claim 23 characterised in that the vehicle is one in which the selected candidate is at least partly soluble and the amount of vehicle and the amount of selected candidate is such that at least some of the selected candidate is dissolved in the said vehicle.